# EUROPEAN PATENT SPECIFICATION

(11) **EP 3 454 784 B1**
(45) Date of publication and mention of the grant of the patent: **01.09.2021**
(21) Application number: 17724470.4
(22) Date of filing: 09.05.2017
(51) Int. Cl.: A61F 2/24, A61F 2/966

(54) **IMPLANT RELEASE SYSTEM**
IMPLANTATFREISETZUNGSSYSTEM
SYSTÈME DE LIBÉRATION D'IMPLANT

(30) Priority: 13.05.2016 US 201662336029 P; 27.04.2017 US 201715499140
(43) Date of publication of application: 20.03.2019
(73) Proprietor: Boston Scientific Scimed, Inc., Maple Grove, MN 55311 (US)
(72) Inventor: WHELTON, Andrew D., Redwood City California 94062 (US); INO, Takashi H., San Jose California 95123 (US); TANAKA, Jonathan M., Los Gatos California 95032 (US); GAMARRA, Randy S., San Jose California 95110 (US); ESCALONA, Floriza Q., San Jose California 95112 (US); MCELLIGOTT, Mark W., Mountain View California 94043 (US); BOYHAN, Noel, Country Westmeath (IE)
(74) Representative: Peterreins Schley
(86) International application number: PCT/US2017/031679
(87) International publication number: WO 2017/196797

(56) References cited:
- WO-A2-2009/134801
- US-A1- 2005 049 674
- US-A1- 2006 259 137
- US-A1- 2010 082 089
- US-A1- 2014 236 278
- US-A1- 2014 303 722

## Description

### Background

Medical devices typically used for cardiovascular system treatments may involve complex and invasive therapies resulting in significant discomfort, pain, and long recovery times for patients. Recently, less invasive, percutaneous treatments have been developed. There is an ongoing need for improved, less invasive cardiovascular treatments.

US 2014/236278 A1 discloses a stent-graft delivery system having a tip capture mechanism. US 2005/049674 A1 discloses a graft body and delivery system. WO 2009/134801 A2 discloses a stent graft delivery system. US 2010/082089 A1 discloses a delivery system for a vascular implant. US 2014/303722 A1 discloses an apparatus for implanting a prosthetic heart valve.

### Summary

The present invention pertains to a medical device release system as set forth in the appended claims.

### Brief Description of the Drawings

The invention may be more completely understood in consideration of the following detailed description of various embodiments of the invention in connection with the accompanying drawings, in which:
Figure 1 is side view of an example medical device system;
Figure 2 is a cross-sectional side view of an example outer sheath;
Figure 3 is a transverse cross-sectional view taken through line 3-3 in Figure 2;
Figure 4 is a side view of an example inner catheter;
Figure 5 is a cross-sectional view taken through line 5-5 in Figure 4;
Figure 6 is a cross-sectional view taken through line 6-6 in Figure 4;
Figure 7 is a perspective view of a portion of an example implant associated with the example medical device system;
Figure 8 is a perspective view of an example release system;
Figure 9 is a perspective view of a release pin;
Figure 10 is a perspective view of a retaining ring;
Figure 11 is a perspective view of a release mandrel;
Figure 12 is a perspective view of the example release system of Figure 8, showing sliding movement between the release pins and the release mandrel;
Figures 13-16 are perspective views that illustrate an example mechanism for locking an implant;
Figure 17 is a side view of a portion of an example sheathing aid;
Figure 18 is an enlarged plan view illustrating engagement of the example sheathing aid with an example implant;
Figure 19 is a side view of an example handle;
Figure 20 is a cut away view illustrating some of the interior components of the example handle;
Figures 21-23 illustrate an example of coordinated movement of handle components within the example handle;
Figures 24-25 illustrate the rotation of a collar on the example handle; and
Figures 26-27 illustrate some of the components within the example handle during rotation of the collar.

While the invention is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives.

### Detailed Description

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (e.g., having the same function or result). In many instances, the term "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The drawings, which are not necessarily to scale, depict illustrative embodiments.

Diseases and/or medical conditions that impact the cardiovascular system are prevalent in the United States and throughout the world. Traditionally, treatment of the cardiovascular system was often conducted by directly accessing the impacted part of the system. For example, treatment of a blockage in one or more of the coronary arteries was traditionally treated using coronary artery bypass surgery. As can be readily appreciated, such therapies are rather invasive to the patient and require significant recovery times and/or treatments. More recently, less invasive therapies have been developed, for example, where a blocked coronary artery could be accessed and treated via a percutaneous catheter (e.g., angioplasty). Such therapies have gained wide acceptance among patients and clinicians.

Some relatively common medical conditions may include or be the result of inefficiency, ineffectiveness, or complete failure of one or more of the valves within the heart. For example, failure of the aortic valve can have a serious effect on a human and could lead to serious health condition and/or death if not dealt with. Treatment of defective heart valves poses other challenges in that the treatment often requires the repair or outright replacement of the defective valve. Such therapies may be highly invasive to the patient. Disclosed herein are medical devices that may be used for delivering a medical device to a portion of the cardiovascular system in order to diagnose, treat, and/or repair the system. At least some of the medical devices disclosed herein may be used to deliver and implant a replacement heart valve (e.g., a replacement aortic valve). In addition, the devices disclosed herein may deliver the replacement heart valve percutaneously and, thus, may be much less invasive to the patient. The devices disclosed herein may also provide a number of additional desirable features and benefits as described in more detail below.

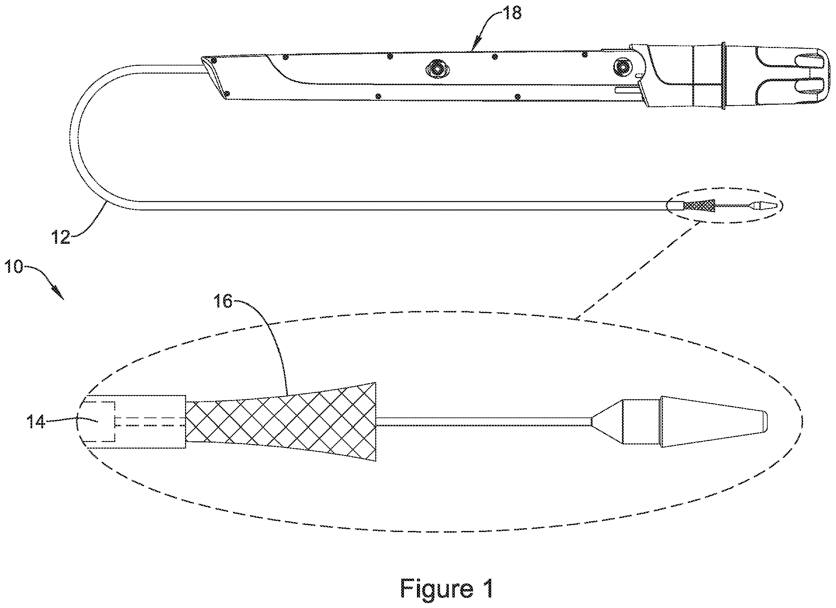

Figure 1 is a side view of an example medical device system 10. It should be noted that some features of system 10 are either not shown, or are shown schematically, in Figure 1 for simplicity. Additional details regarding some of the components of system 10 are provided in other figures in greater detail. System 10 may be used to deliver and/or deploy a variety of medical devices to a number of locations within the anatomy. In at least some embodiments, system 10 is a replacement heart valve delivery system (e.g., a replacement aortic valve delivery system) that can be used for percutaneous delivery of a replacement heart valve. This, however, is not intended to be limiting as system 10 may also be used for other interventions including mitral valve replacement, valve repair, valvuloplasty, and the like, or other similar interventions.

System 10 may generally be described as a catheter system that includes a catheter or outer sheath 12 and tube or inner catheter 14 (a portion of which is shown in Figure 1 in phantom line) extending at least partially through outer sheath 12. A medical device implant 16 may be coupled to inner catheter 14 and disposed within outer sheath 12 during delivery of implant 16. A handle 18 may be disposed at the proximal end of outer sheath 12 and inner catheter 14. In general, handle 18 may be configured to manipulate the position of outer sheath 12 relative to inner catheter 14 as well as aid in the deployment of implant 16.

In use, system 10 may be advanced percutaneously through the vasculature to a position adjacent to an area of interest. For example, system 10 may be advanced through the vasculature to a position adjacent to a defective aortic valve. During delivery, implant 16 may be generally disposed in an elongated and low profile "delivery" configuration within outer sheath 12. Once positioned, outer sheath 12 may be retracted to expose implant 16. Implant 16 may be actuated in order to expand implant into a generally shortened and larger profile "deployed" configuration suitable for implantation within the anatomy. When implant 16 is suitably deployed within the anatomy, system 10 can be removed from the vasculature, leaving implant 16 in place to function as, for example, a suitable replacement for the native aortic valve. In at least some interventions, implant 16 may be deployed within the native valve (e.g., the native valve is left in place and not excised). Alternatively, the native valve may be removed and implant 16 may be deployed in its place as a replacement.

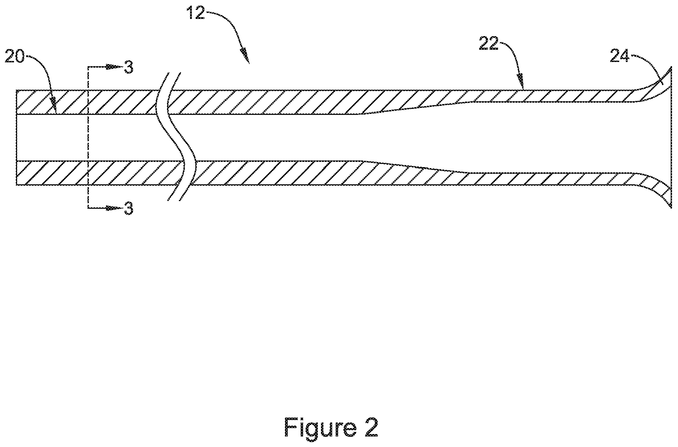

Figures 2-18 (as well as other figures) illustrate some of the components of system 10. For example, Figure 2 is a cross-sectional side view of outer sheath 12. Here it can be seen that outer sheath 12 has a proximal portion 20 and a distal portion 22. Distal portion 22 may have a slightly enlarged or flared inner diameter, which may provide additional space for holding implant 16 therein. For example, the inner diameter of outer sheath 12 along proximal portion 20 may be in the range of about 0.254 to 1.27 cm (0.10 to 0.50 inches), or about 0.508 to 1.016 cm (0.20 to 0.40 inches), or about 0.508 to 0.762 cm (0.20 to 0.30 inches), or about 0.56388 ± 0.0508 cm (0.222 ± 0.002 inches). The inner diameter of outer sheath 12 along distal portion 22 may be in the range of about 0.254 to 1.27 cm (0.10 to 0.50 inches), or about 0.508 to 1.016 cm (0.20 to 0.40 inches), or about 0.508 to 0.762 cm (0.20 to 0.30 inches), or about 0.579 to 0.5842 cm (0.228 to 0.230 inches). At the distal end of distal portion 22 may be a distal tip 24, which may be flared or otherwise have a funnel-like shape. The funnel-like shape increases the outer diameter (and inner diameter) of outer sheath 12 at distal tip 24 and may aid in the sheathing and/or re-sheathing of implant 16 into outer sheath 12. Other than at distal tip 24, outer sheath 12 may have a generally constant outer diameter. For example, outer sheath 12 may have an outer diameter in the range of about 0.254 to 1.27 cm (0.10 to 0.50 inches), or about 0.508 to 1.016 cm (0.20 to 0.40 inches), or about 0.508 to 0.762 cm (0.20 to 0.30 inches), or about 0.6858 cm (0.270 inches). These are just examples. Other embodiments are contemplated that have differing dimensions (including those appropriate for differently sized patients, including children) and/or arrangements for the outer diameter and/or inner diameter of outer sheath 12. These contemplated embodiments include outer sheaths 12 with flared or otherwise variable outer diameters, embodiments with constant inner diameters, combinations thereof, and the like. Outer sheath 12 may also have a length that is appropriate for reaching the intended area of interest within the anatomy. For example, outer sheath 12 may have a length in the range of about 30 to 200 cm, or about 60 to 150 cm, or about 100 to 120 cm, or about 108 ± 0.20 cm. Outer sheath 12 may also be curved. For example, a distal section of outer sheath 12 may be curved. In one example, the radius of the curve (measured from the center of outer sheath 12) may be in the range of about 2 to 6 cm (20 to 60 mm), or about 3 to 4 cm (30 to 40 mm), or about 3.675 cm (36.75 mm). Again, these dimensions are examples and are not intended to be limiting.

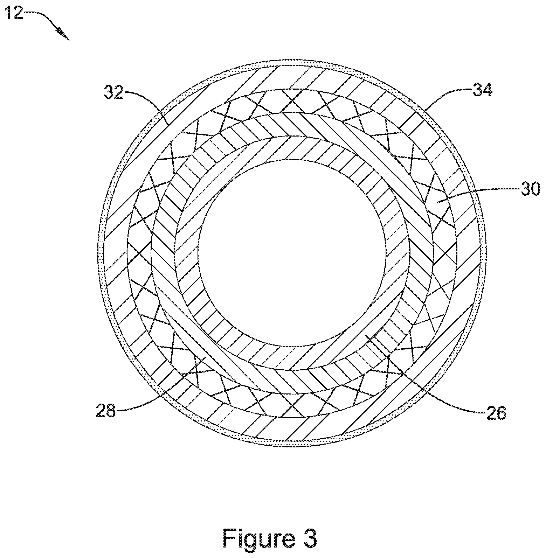

Outer sheath 12 may be formed from a singular monolithic tube or unitary member. Alternatively, outer sheath 12 may include a plurality of layers or portions. One or more of these layers may include a reinforcing structure such as a braid, coil, mesh, combinations thereof, or the like. Figure 3 illustrates one example of a multilayer structure for outer sheath 12. For example, outer sheath 12 may include a layer or inner liner 26. A tier layer or intermediate layer 28 may be disposed on inner liner 26. A reinforcement 30 may be disposed on intermediate layer 28. An outer layer or topcoat 32 may be disposed on reinforcement 30. Further, an outer coating 34 (e.g., a lubricious coating, a hydrophilic coating, a hydrophobic coating, etc.) may be disposed along portions or all of topcoat 32. These are just examples. Several alternative structural configurations are contemplated for outer sheath 12 including embodiments including two or more layers that may be different from those shown in Figure 3, embodiments without a reinforcement, and the like, or other suitable configurations.

The dimensions and materials utilized for the various layers of outer sheath 12 may also vary. For example, inner liner 26 may include a polymeric material such as fluorinated ethylene propylene (FEP) and may have a thickness in the range of about 0.00254 to 0.0127 cm (0.001 to 0.005 inches) or about 0.00762 ± 0.00254 (0.003 ± 0.001 inches), intermediate layer 28 may include a polymer material such as polyether block amide (e.g., PEBAX 6333) and may have a thickness in the range of about 0.00254 to 0.0127 cm (0.001 to 0.005 inches) or about 0.00508 ± 0.00254 (0.002 ± 0.001 inches), outer coating 34 may include a polymer material such as polyether block amide (e.g., PEBAX 7233) and may have a thickness in the range of about 0.00254 to 0.0254 cm (0.001 to 0.01 inches). In some embodiments, outer coating 34 may vary in thickness. For example, along proximal portion 20 outer coating 34 may have greater thickness, such as about 0.0127 to about 0.0508 cm or about 0.02159 cm (0.005 to 0.02 inches or about 0.0085 inches), than along distal portion 22 and/or distal tip 24, which may be about 0.0127 to about 0.0508 cm or about 0.01651 cm (e.g., about 0.005 to 0.02 inches or about 0.0065 inches). These are just examples as other suitable materials may be used.

The form of distal tip 24 may also vary. For example, in at least some embodiments, inner liner 26 (e.g., a 2.5 mm section thereof) may be extended up and around the distal end of outer sheath 12 (e.g., around reinforcement 30 and topcoat 32). A ring member (not shown) made from a suitable material such as a 55D polyether block amide (e.g., 55D PEBAX) may be disposed over inner liner 26 and heat bonded to form distal tip 24. This may form the funnel-like shape of distal tip 24.

Reinforcement 30 may also vary in form. In at least some embodiments, reinforcement 30 may take the form of a braid, coil, mesh, or the like. For example, in some embodiments, reinforcement 30 may include a metallic braid (e.g., stainless steel). In some of these embodiments, reinforcement 30 may also include additional structures such as one or more longitudinally-extending strands. For example, reinforcement 30 may include a pair of longitudinally-extending aramid and/or para aramid strands (for example, KEVLAR®) disposed on opposite sides of the braid. These strands may or may not be woven into portions or all of the braid.

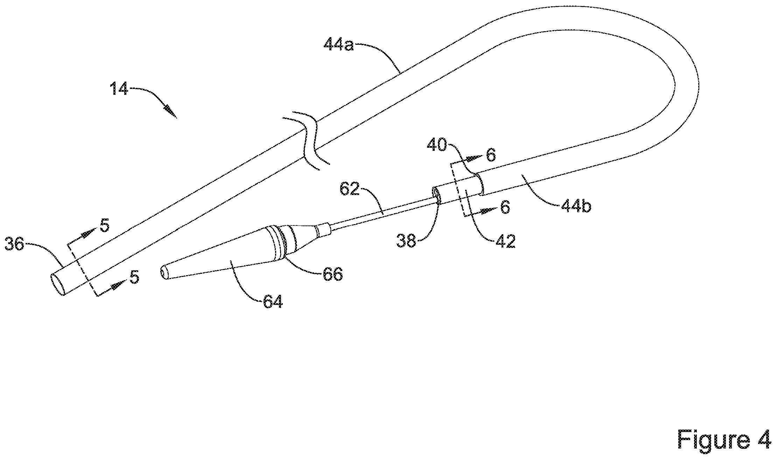

Figure 4 is a side view of the inner catheter 14. A distal end region of inner catheter 14 may include a step in outer diameter 40 that defines a decreased outer diameter section 42. For example, decreased outer diameter section 42 may have an outer diameter in the range of about 0.127 to 0.635 cm (0.05 to 0.25 inches), or about 0.254 to 0.508 cm (0.10 to 0.20 inches), or about 0.38608 ± 0.00762 (0.152 ± 0.003 inches) as opposed to the remainder of inner catheter 14 where the outer diameter may be in the range of about 0.127 to 0.762 cm (0.05 to 0.30 inches), or about 0.254 to 0.635 cm (0.10 to 0.25 inches), or about 0.508 ± 0.0254 cm (0.20 ± 0.01 inches). Decreased outer diameter section 42 may define a region where other components of system 10 may be attached. Some additional details regarding these components can be found herein.

In general, inner catheter 14 may take the form of an extruded polymer tube. Other forms are also contemplated including other polymer tubes, metallic tubes, reinforced tubes, or the like including other suitable materials such as those disclosed herein. In some embodiments, inner catheter 14 is a singular monolithic or unitary member. In other embodiments, inner catheter 14 may include a plurality of portions or segments that are coupled together. The total length of inner catheter may be in the range of about 60 to 150 cm, or about 80 to 120 cm, or about 100 to 115 cm, or about 112 ± 0.02 cm. Just like outer sheath 12, inner catheter 14 may also be curved, for example adjacent to the distal end thereof. In some embodiments, inner catheter 14 may have one or more sections with a differing hardness/stiffness (e.g., differing shore durometer). For example, inner catheter may have a proximal region 44a and an intermediate region 44b. Proximal region 44a may include a generally stiff polymeric material such as a 72D polyether block amide (e.g., 72D PEBAX) and may have a length in the range of about 60 to 150 cm, or about 80 to 120 cm, or about 100 to 115 cm, or about 109.5 ± 0.02 cm. Intermediate region 44b may include a 40D polyether block amide (e.g., 40D PEBAX) and may have a length in the range of about 5 to 25 mm, or about 10 to 20 mm, or about 15 ± 0.01 mm. Decreased outer diameter section 42 may also differ from regions 44a/44b and, in some embodiments, may include a 72D polyether block amide (e.g., 72D PEBAX) and may have a length in the range of about 0.5 to 2 cm (5 to 20 mm), or about 0.8 to 1.5 cm (8 to 15 mm), or about 1 ± 0.001 cm (10 ± 0.01 mm). These are just examples.

Inner catheter 14 may also include a guidewire extension tube 62 that extends distally from distal end portion 38. A nose cone 64 is attached to guidewire extension tube 62. Nose cone 64 generally is designed to have an atraumatic shape. Nose cone 64 may also include a ridge or ledge 66 that is configured to abut the distal tip 24 of outer sheath 12 during delivery of implant 16.

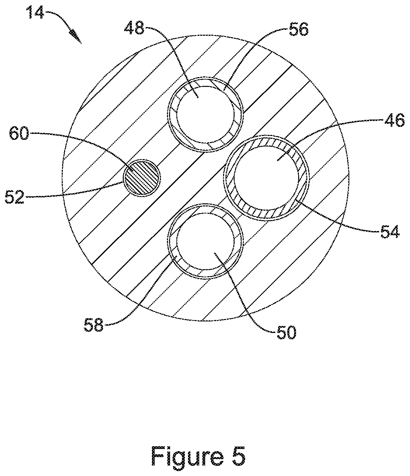

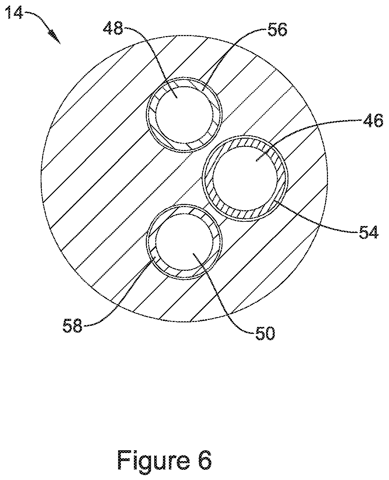

Inner catheter 14 may include one or more lumens. For example, Figure 5 (which is a cross-sectional view of inner catheter 14 adjacent to proximal end portion 36) illustrates that inner catheter 14 may include a first lumen 46, a second lumen 48, a third lumen 50, and a fourth lumen 52. In general, lumens 46,48,50,52 extend along the entire length of inner catheter 14. Other embodiments are contemplated, however, where one or more of lumens 46,48,50,52 extend along only a portion of the length of inner catheter 14. For example, fourth lumen 52 may stop just short of the distal end of inner catheter 14 and/or be filled in at its distal end to effectively end fourth lumen 52 proximal of the distal end of inner catheter 14, as illustrated in Figure 6 by the absence of fourth lumen 52 adjacent to the distal end of inner catheter 14.

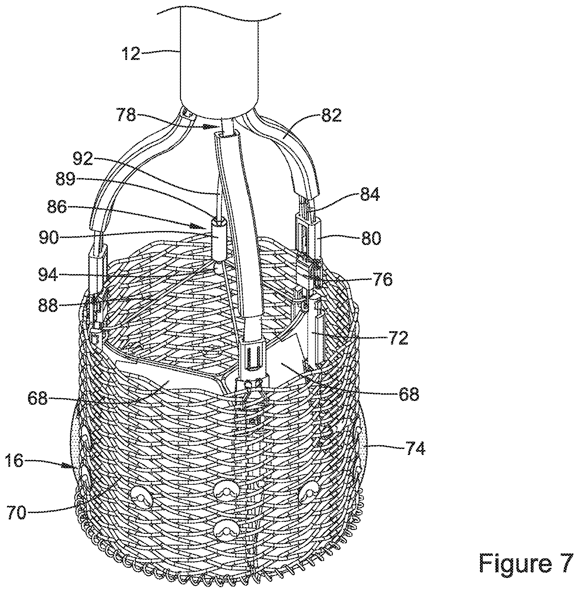

Disposed within first lumen 46 may be push-pull rods 84 (not shown in Figure 5, seen in Figure 7), which are used to expand and/or elongate implant 16 as explained in more detail herein. In at least some embodiments, first lumen 46 may be lined with a low friction liner 54 (e.g., a FEP liner). Disposed within second lumen 48 may be a pin release mandrel 92 (not shown in Figure 5, seen in other figures including Figure 7), which is also explained in more detail herein. In at least some embodiments, second lumen 48 may be lined with a hypotube liner 56. Third lumen 50 may be a guidewire lumen and this lumen may also be lined with a hypotube liner 58.

Fourth lumen 52 may be used to house a non-stretch wire 60. The form of non-stretch wire 60 may vary. In some embodiments, non-stretch wire 60 may take the form of a stainless steel braid. Non-stretch wire 60 may optionally include a pair of longitudinally-extending aramid and/or para aramid strands (for example, KEVLAR®) disposed on opposite sides of the braid. In general, rather than being "disposed within" fourth lumen 52, non-stretch wire 60 may be embedded within fourth lumen 52. In addition, non-stretch wire 60 may extend to a position adjacent to distal end portion 38 but not fully to the distal end of inner catheter 14 as illustrated in Figure 6 by the absence of fourth lumen 52 adjacent to the distal end of inner catheter 14. For example, a short distal segment of fourth lumen 52 may be filled in with polymer material adjacent to the distal end of inner catheter 14.

Figure 7 illustrates some of the additional components of system 10 and implant 16. For example, here it can be seen that implant 16 includes a plurality of valve leaflets 68 (e.g., bovine pericardial) which are secured to a cylindrical braid 70 at a post or commissure post 72, for example at the commissure portions of the leaflets 68. In this example, implant 16 includes three leaflets 68 secured to braid 70 with three posts 72. Leaflets 68 may also be secured to the base or "distal end" of braid 70. The posts 72, in turn, may be secured to braid 70 (e.g., along the interior of braid 70) with sutures or other suitable mechanisms. Positioned adjacent to (e.g., longitudinally spaced from and aligned with) posts 72 are a plurality of buckles 76, which may also be sutured to braid 70 (e.g., along the interior of braid 70). In this example, one buckle 76 is attached to braid 70 adjacent to each of the three posts 72. Accordingly, braid 70 has a total of three buckles 76 and three posts 72 attached thereto. Other embodiments are contemplated where fewer or more buckles 76 and posts 72 may be utilized. A seal 74 (shown in cross-section) may be disposed about braid 70 and, as the name suggests, may help to seal implant 16 within a target implant site or area of interest.

Attachment between implant 16 and inner catheter 14 (and/or outer sheath 12) may be effected through the use of a three finger coupler 78. Coupler 78 may generally include a cylindrical base (not shown) that is attached to inner catheter 14 (e.g., disposed about and attached to reduced outer diameter section 42). Projecting distally from the base are three fingers that are each configured to engage with implant 16 at posts 72 and buckles 76. A collar 80 may further assist in holding together these structures. A guide 82 may be disposed over each of the fingers and may serve to keep the fingers of coupler 78 associated with push-pull rods 84 extending adjacent to coupler 78. Finally, a pin release assembly 86 may be a linking structure that keeps posts 72, buckles 76, and push-pull rods 84 associated with one another.

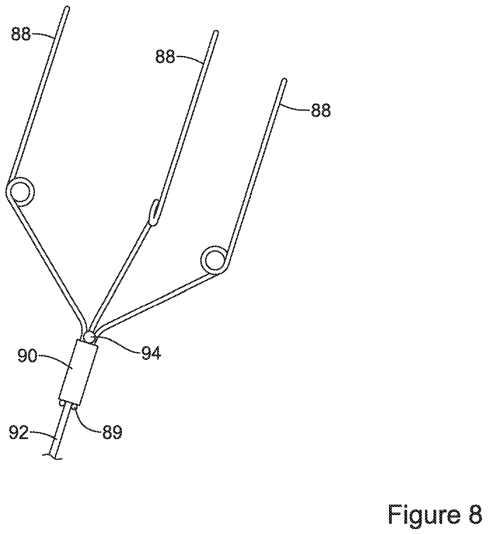

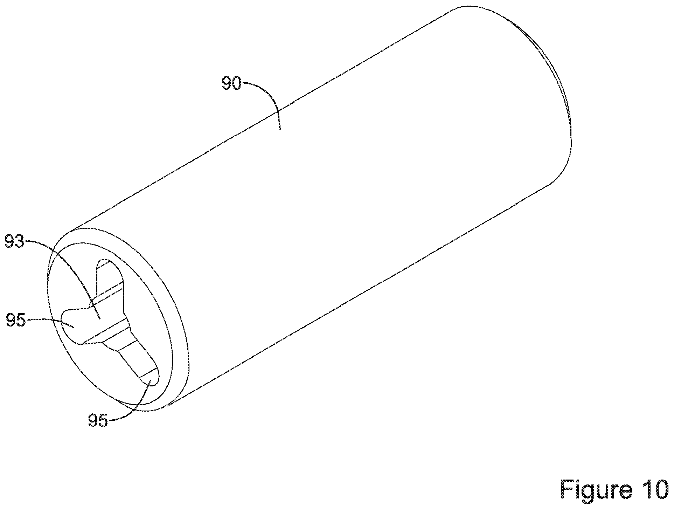

Pin release assembly 86 includes a plurality of individual pins 88 that may be joined together via a retaining ring 90 and held in place by an enlarged distal end 94. As shown in Figure 8, the enlarged distal end 94 on the release mandrel 92 prevents the retaining ring 90 from sliding off the distal end of the mandrel 92. The individual pins 88 may be joined together by having their proximal ends welded to the retaining ring 90. The retaining ring 90 has a center channel 93 and side channels 95, which preferably extend longitudinally therethrough, as shown in Figure 10. The number of side channels 95 may correspond with the number of pins 88. The side channels 95 may be separated from the center channel 93, or the side channels 95 may extend radially from the center channel 93. The retaining ring shown in Figure 10 has three side channels 95 extending radially from the center channel 93 and receives three pins 88. The three pins 88 may be spaced at 120 degree intervals around the ring. The pins 88 may be made of a metallic material, such as nickel-titanium alloy, stainless steel, and the like, or other suitable corrosion-resistant materials.

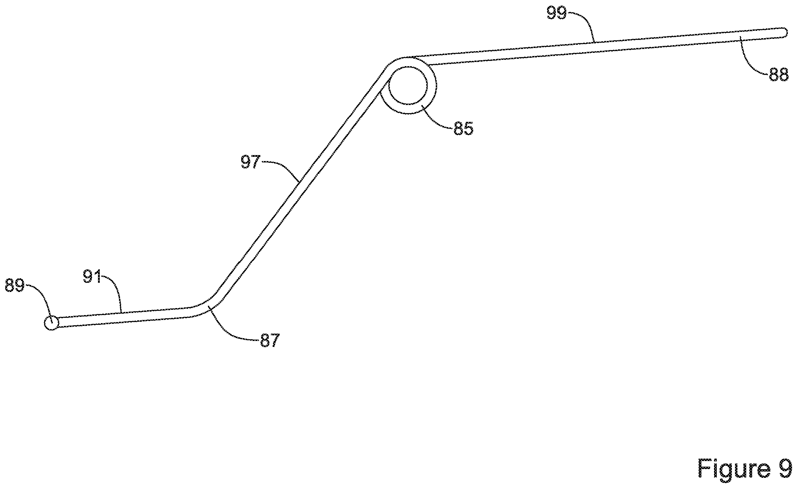

The pins 88 may be disposed through the side channels 95, with an enlarged proximal tip 89 on each pin extending beyond the proximal end of the retaining ring 90. The enlarged proximal tip 89 is larger than an inner diameter of the side channel 95, preventing the pin 88 from being pulled through the retaining ring in the proximal direction. As shown in Figure 9, the pins 88 may have a first bend 87 formed therein as the pins exit the distal end of the retaining ring 90. The combination of the enlarged proximal tip 89 and the first bend 87 of the pins 88 may hold the pins in a fixed axial position within the retaining ring 90. In some embodiments, the pins 88 may freely rotate within the side channels 95. In other embodiments, the inner diameter of the side channels 95 provides a friction fit with the pins 88, thereby restricting or preventing rotational as well as axial movement of the pins 88 within the retaining ring 90. Alternatively, or additionally, the pins 88 may be welded to the retaining ring or attached with adhesive.

Each pin has a first section 91 disposed between the enlarged proximal tip 89 and the first bend 87, a second section 97 extending from the first bend 87 to a second bend 85, and a third section 99 extending from the second bend 85 to the distal end. At the second bend 85, the third section 99 may be angled from the second section 97 by between 90 and 140 degrees, such as 115 degrees. In some embodiments, the second bend 85 is around 360 degrees or greater than 360 degrees, such as between 450 and 500 degrees, forming a spring. The pins shown in FIGS. 8 and 9 have second bend 85 of about 475 degrees forming a single loop spring between the second section 97 and third section 99. The spring may provide stress relief between the implant 16 and the pin release mandrel 92.

The retaining ring 90 is slidably disposed on the distal end of the pin release mandrel 92. The release mandrel 92 may be made of a metallic material, such as Elgiloy®, nickcl-titanium alloy, stainless steel, and the like, or other suitable corrosion-resistant materials. The release mandrel 92 may be formed by drawing a straight wire followed by forming a ball on the tip. No grinding of the mandrel 92 is necessary.

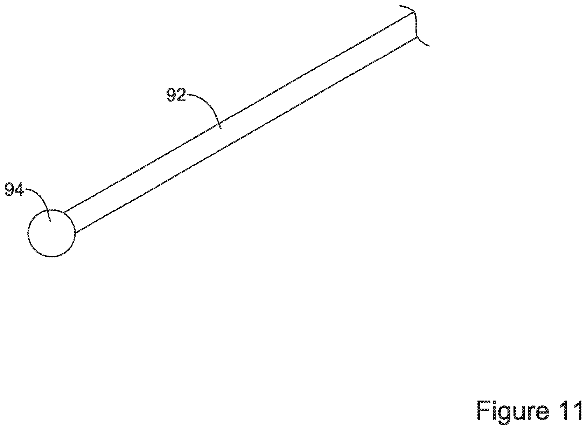

The release mandrel 92 is slidably disposed within the center channel 93 of the retaining ring 90, allowing the retaining ring and pin assembly 86 to move independently of the release mandrel. As shown in detail in Figure 11, an enlarged distal end 94 on the release mandrel 92 prevents the retaining ring 90 from sliding off the distal end of the mandrel.

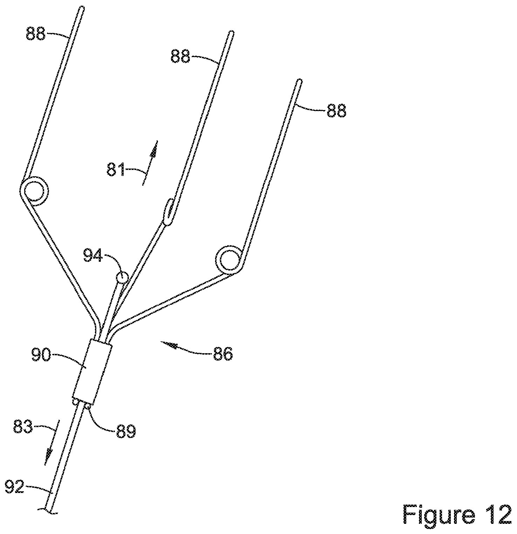

The sliding engagement of the retaining ring 90 on the release mandrel 92 provides a floating pin assembly 86, as shown in Figure 12. As the implant structure is drawn into the inner catheter 14 in preparation for delivery, the implant and the release assembly 86 are compressed. The retaining ring 90 and the release mandrel 92 slide relative to each other, providing independent movement and reducing stress throughout the catheter system. The retaining ring 90 slides relative to the release mandrel 92 in a proximal direction 83 and a distal direction 81. Additionally, as the entire system is guided through the body, the sliding relationship between the retaining ring and the release mandrel reduces the transmission of torque from the proximal end of the release mandrel to the pins. Reducing stress on the pin release assembly 86 may prevent damage to the release assembly and release mandrel.

The proximal end of the release mandrel 92 extends proximally through the inner catheter 14. The release mandrel 92 may be withdrawn proximally 83 to release the pins 88 distally 81. The enlarged distal end 94 of the release mandrel 92 is larger than an inner diameter of the center channel 93, preventing the retaining ring 90 from sliding off the enlarged distal end 94 of the release mandrel 92. The enlarged distal end 94 engages the retaining ring 90, thus proximal movement of the release mandrel 92 will be translated to proximal movement of retaining ring and pins 88, thereby removing the pins 88 from the push-pull rods 84 and posts 72 assembly, as described below.

The sliding retaining ring 90 may provide a reduced profile attachment of the pins 88 to the release mandrel 92. The retaining ring 90 may have an outer diameter of about 0.053 inch [1.3462 mm], reduced by up to 0.014 inch [0.3556 mm] compared to systems in which proximal ends of release pins are wrapped or coiled around the release mandrel and welded in place. This is a significant reduction in the outer diameter, and provides an easier assembly process.

The enlarged proximal tips 89 on the pins 88 and the enlarged distal end 94 on the release mandrel 92, as well as any welding of the pins 88 to the retaining ring 90 may be formed, for example, by using a suitable welding method such as laser welding, GTAW (TIG) welding, spot welding, and the like.

During delivery, implant 16 is secured at the distal end of inner catheter 14 by virtue of the association of the fingers of coupler 78 being coupled with a projecting proximal end of buckles 76 (and being held in place with collar 80 disposed over the connection) and by virtue of pins 88 securing together push-pull rods 84 and posts 72. When implant 16 is advanced within the anatomy to the desired location, outer sheath 12 may be withdrawn (e.g., moved proximally relative to inner catheter 14) to expose implant 16. Then, push-pull rods 84 can be used to expand and "lock" implant 16 in the expanded or deployed configuration by proximally retracting push-pull rods 84 to pull posts 72 into engagement with buckles 76. Finally, pins 88 can be removed, thereby uncoupling push-pull rods 84 from posts 72, which allows implant 16 to be released from system 10 and deployed in the anatomy.

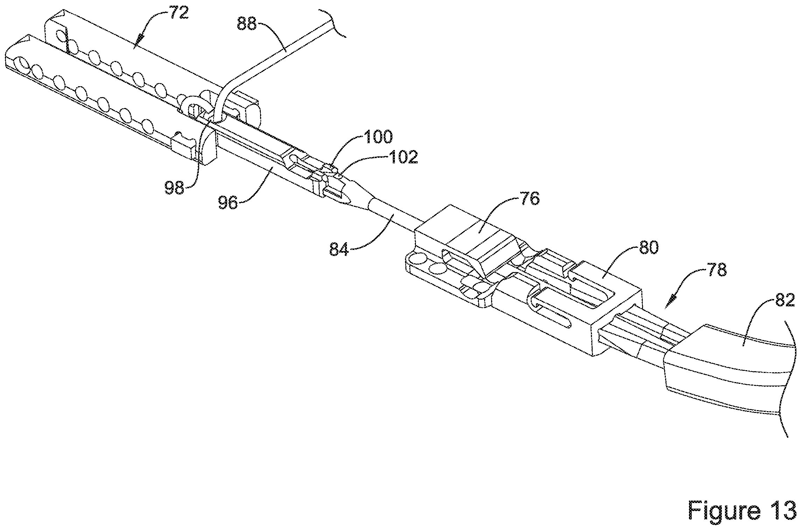

Figures 13-16 illustrate the locking system utilized with system 10. For simplicity purposes, only one of the three fingers of the coupler 78, only one of the three push-pull rods 84, and only one of the posts 72 of the example system 10 are shown (and implant 16 is not shown). As seen in Figure 13, push-pull rod 84 extends through guide 82 adjacent to the fingers of coupler 78, through collar 80, through buckle 76, and into a hollow t-shaped bar portion 96 of post 72. The distal end of push-pull rod 84 may include an opening or aperture (not shown) that can be aligned with an opening 98 of t-shaped bar portion 96. When so aligned, pin 88 can be looped through opening 98 and the opening of push-pull rod 84. This secures push-pull rod 84 to post 72 and forms a configuration of these structures that can be utilized during delivery of implant 16. As can be appreciated, the proximal end of post 72 and the distal end of buckle 76 are longitudinally separated and, accordingly, implant 16 is in an elongated and generally low-profile configuration suitable for delivery.

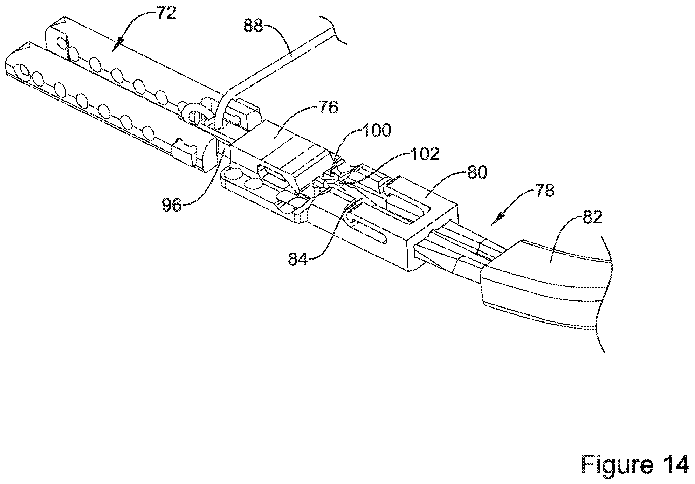

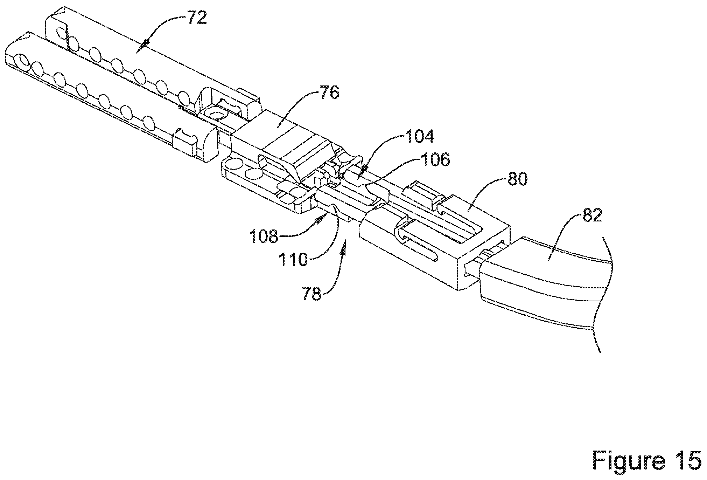

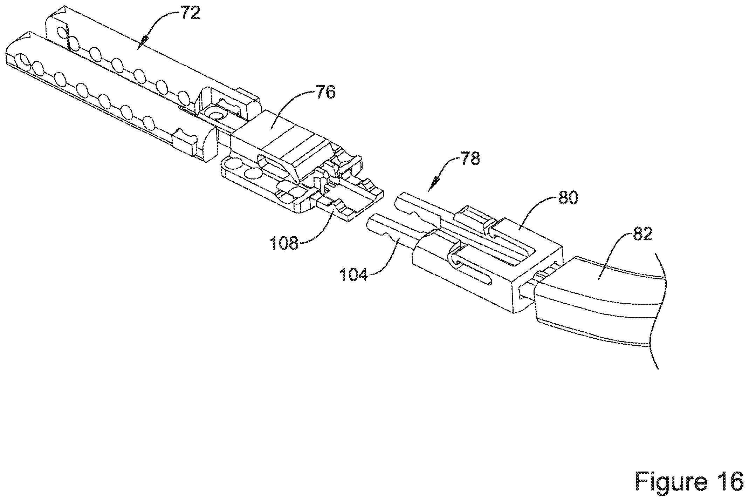

When implant 16 reaches the intended target site within the anatomy, a clinician can proximally retract push-pull rod 84, thereby moving the proximal ends of posts 72 toward the distal ends of buckles 76 in order to expand implant 16. Ultimately, push-pull rod 84 can be retracted sufficiently far enough to lock post 72 with buckle 76 so as to lock implant in an expanded configuration suitable for implantation within the anatomy. Figure 14 illustrates push-pull rod 84 proximally rctractcd. In doing so, post 72 is brought into contact with buckle 76. More particularly, a raised, generally transversely-oriented ridge 100 on t-shaped bar portion 96 may be pulled proximally past buckle 76 so that post 72 is secured and held in place by buckle 76. At this point, it is possible to urge push-pull rods 84 distally to "unlock" implant 16, thereby allowing for repositioning and/or retraction. Alternatively, if a clinician is satisfied with the positioning and/or locking of implant 16 (e.g., after visualization of implant 16 via a suitable imaging technique), pins 88 may be pulled (e.g., removed from openings 98 and the openings in push-pull rods 84) to uncouple push-pull rods 84 from posts 72 as shown in Figure 15. Further retraction of push-pull rods 84 causes a longitudinally-oriented ridge 102 on push-pull rods 84 to engage collar 80 and causes collar 80 to slide proximally along the fingers of coupler 78. In doing so, a forked end 104 of the fingers, which has a groove 106 formed therein, is exposed and can be uncoupled from a rail 108, which has a projection 110 formed thereon that is configured to mate with groove 106, as shown in Figure 16. Thereafter, system 10 can be removed from the anatomy, leaving behind the expanded and deployed implant 16.

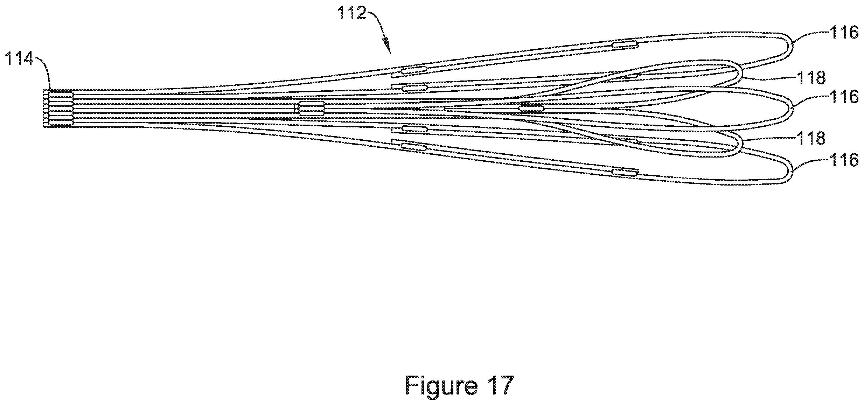

Figures 17-18 illustrate another component that may be included with system 10. For example, Figure 17 is a side view of a portion of a sheathing aid 112. Here it can be seen that sheathing aid 112 includes a base 114 and a group of petals including a set of three longer petals 116 and a pair of shorter petals 118. In use, a group of petals 116/118 may be positioned between each of the fingers of coupler 78. Because the coupler 78 may have a total of three fingers, sheathing aid 112 may have a total of fifteen petals (e.g., three groups that each include three "long" petals 116 and two "short" petals 118, with each group being positioned between adjacent pairs of fingers of coupler 78). Base 114 may be secured to inner catheter 14 adjacent to coupler 78 (e.g., underneath coupler 78 and between coupler 78 and inner catheter 14).

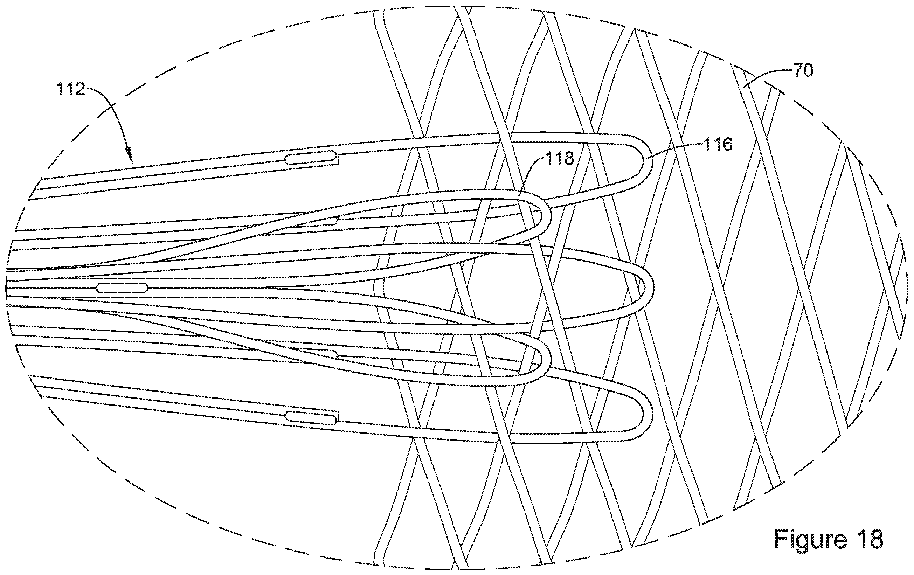

Sheathing aid 112, as the name suggests, may be used to aid in the sheathing of implant 16 into outer sheath 12. In addition, sheathing aid 112 may aid in the initial sheathing of implant 16 (e.g., removing implant 16 from a packaging container such as a bottle and pulling implant 16 into outer sheath 12) and in re-sheathing implant 16 during repositioning and/or retraction of implant 16 within the area of interest. Sheathing may be accomplished via the arrangement and positioning of the various petals 116/118. For example, Figure 18 illustrates the longer petals 116 woven in and out of braid 70, and the shorter petals 118 disposed along the exterior of braid 70 acting as a funnel for sheathing.

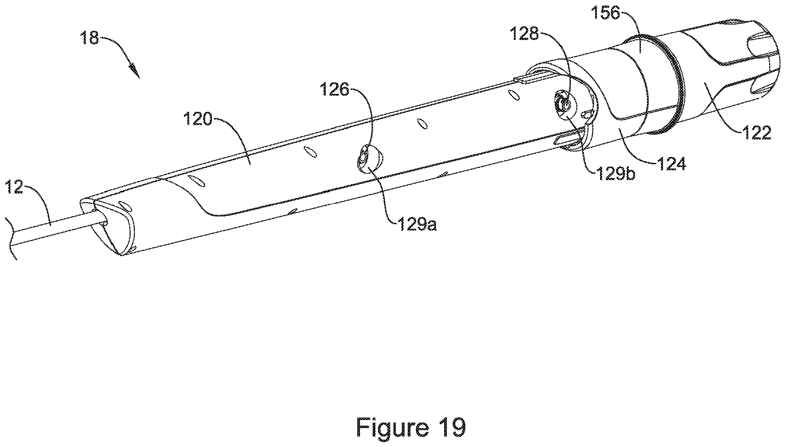

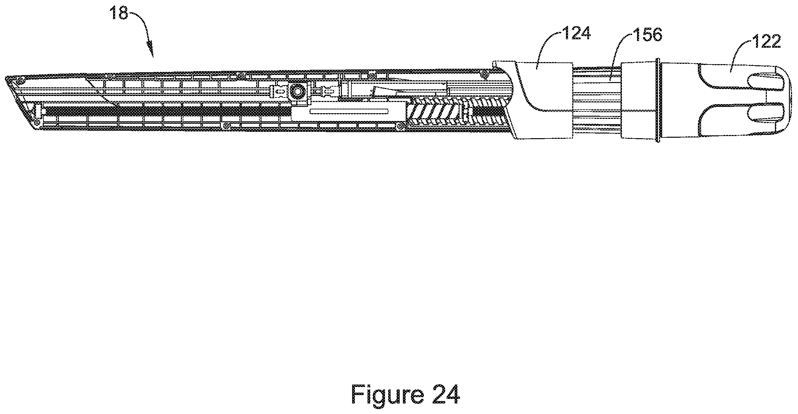

Figure 19 is a side view of handle 18. Here it can be seen that handle 18 includes a handle housing 120. A rotatable control knob 122 may be disposed about handle housing 120 (e.g., at a proximal end of handle housing 120) and may be used to move one or more of the components of system 10 (e.g., outer sheath 12, push-pull rods 84, etc.). A rotatable collar 156 may be disposed about the handle housing 120. In some embodiments, control knob 122 may be disposed about a proximal portion of collar 156. A slidable door 124 may also be disposed about handle housing 120. Door 124 may translate distally to expose a distal portion of rotatable collar 156 (not shown in Figure 19, can be seen in other figures including Figures 24-25) positioned generally under door 124. Collar 156 may be rotated to move one or more components of system 10 (e.g., push-pull rods 84, pin release mandrel 92, etc.). Handle 18 may also include one or more apertures 129a/129b and/or flush ports 126/128 that can be used to flush system 10. In some embodiments, distal flush port 126 and proximal flush port 128 may be accessible from the exterior of the handle housing 120 through distal aperture 129a and proximal aperture 129b, respectively.

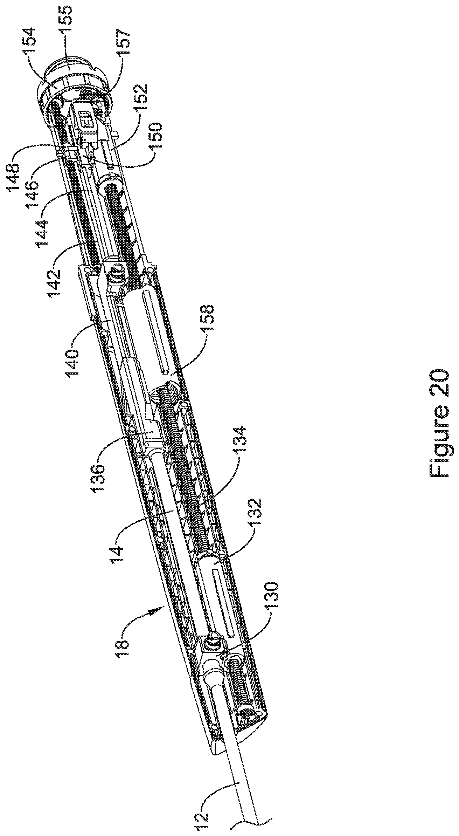

Figure 20 is a side view of handle 18 with a portion of handle housing 120 removed, exposing at least some of the interior components. Here it can be seen that outer sheath 12 may be attached to a sheath adapter 130. Sheath adapter 130 is attached to a sheath carriage 132, which may be threaded onto a lead screw 134. Distal flush port 126 may be disposed on sheath adapter 130. In general, distal flush port 126 provides access to the interior or lumen of outer sheath 12 (e.g., access to space between inner catheter 14 and outer sheath 12) so that a clinician can flush fluid through the lumen of outer sheath 12 to remove any unwanted materials (e.g., air, fluid, contaminants, etc.) therein prior to use of system 10. In at least some embodiments, distal flush port 126 has a luer type connector (e.g., a one-way luer connector) that allows a device such as a syringe with a corresponding connector to be attached thereto for flushing.

Extending through and proximally from sheath adapter 130 is inner catheter 14. A proximal end of inner catheter 14 is attached (e.g., fixedly attached) to an interior body or diverter 136. Diverter 136 is attached to a support body 140. In general, diverter 136 and/or support body 140 may have one or more passageways or lumens formed therein. In some embodiments, push-pull rods 84 and/or pin release mandrel 92 may extend through respective passageways. Alternatively, the proximal ends of push-pull rods 84 and/or pin release mandrel 92 may each be attached to a shaft or hypotube (e.g., solid in cross-section, tubular, etc.), and each of the shafts may extend through the one or more passageways. For example, a first shaft or hypotube 142 and a second shaft or hypotube 144 may extend through the passageways in diverter 136, and in some embodiments, the first shaft or hypotube 142 extends through a first passageway and the second shaft or hypotube 144 extends through a second passageway that is separate or distinct from the first passageway. In at least some embodiments, first shaft 142 is attached to pin release mandrel 92. In at least some embodiments, second shaft 144 is attached to push-pull rods 84. It should be noted that at in least some embodiments of system 10, three push-pull rods 84 are utilized. In these embodiments, the three push-pull rods 84 come together (e.g., brought into contact with one another or otherwise brought into relatively close proximity with one another) adjacent to the distal end of inner catheter 14 and enter first lumen 46. At one or more positions along their length, push-pull rods 84 may be attached to one another. For example, in some embodiments, push-pull rods 84 may be welded together about 10.16 cm (about 4.00 inches) from their distal ends. In some embodiments, push-pull rods 84 may be welded together proximate their proximal ends in addition to or instead of the distal weld. Proximally thereafter, push-pull rods 84 may extend to second shaft 144.

A hypotube (e.g., hypotube liner 58 disposed along guidewire lumen 52) may extend through diverter 136 within a passageway therein and then be "diverted" around a portion of diverter 136 and support body 140, and ultimately be extended to a position at the proximal end of handle 18 so as to provide a user access to guidewire lumen 52. Proximal flush port 128 may be disposed on support body 140 that can be used to flush the lumens of inner catheter 14 and, for example, may function similarly to distal flush port 126.

At their respective proximal ends, first shaft 142 may be secured to a slider 146 and second shaft 144 may be secured to a force limiter body 150. The connections between the various components may include a number of different types of connections including mechanical bonding (e.g., pinning, threading, interference fit, etc.), adhesive bonding, thermal bonding, etc. Slider 146 may be slidable relative to force limiter body 150. In some embodiments, slider 146 may be selectively locked to force limiter body 150, thereby preventing relative movement between the slider 146 and the force limiter body 150. Force limiter body 150 may be secured to a push-pull rod carriage 152, which may be threaded onto lead screw 134. Thus, movement of lead screw 134 can cause movement of push-pull rod carriage 152 and force limiter body 150 and thus, push-pull rods 84 (via second shaft 144). Some additional details regarding this motion can be found herein.

In general, force limiter body 150 forms or defines a stop point that provides tactile feedback (e.g., resistance to further rotation of control knob 122) to the user indicating that push-pull rods 84 have been retracted proximally a sufficient distance to lock posts 72 with buckles 76. To verify proper locking, a clinician may use an appropriate visualization technique to visualize proper locking (e.g., the relative positioning of the posts 72 and the buckles 76). A chock 148 may be positioned adjacent to slider 146 to selectively lock slider 146 to force limiter body 150. In order to allow pin release mandrel 92 to be proximally retracted to pull pins 88, chock 148 can be rotated or otherwise moved to a secondary position or configuration. When in this configuration, chock 148 no longer forms a barrier to further movement of, for example, slider 146 and pin release mandrel 92. Accordingly, with chock 148 no longer acting as an impediment, slider 146 and pin release mandrel 92 can be proximally retracted to facilitate deployment of implant 16 by allowing pins 88 to be pulled.

Handle 18 also includes a rotatable ring 155 with internal teeth that are configured to engage with teeth on a gear 157 coupled to lead screw 134. Ring 155 is coupled to control knob 122 so that rotation of control knob 122 results in analogous motion of ring 155 and thus lead screw 134.

Handle 18 is generally configured for coordinated movement of multiple structures of system 10. For example, handle 18 is configured to allow a user to move outer sheath 12 (e.g., relative to inner catheter 14), move push-pull rods 84, and move pin release mandrel 92. Moreover, handle 18 is configured so that the appropriate structure can be moved at the appropriate time during the intervention so that implant 16 can be delivered in an efficient manner. Some examples of how the coordinated movement of system 10 may occur within handle 18 may be similar to those disclosed in U.S. Patent Application Pub. No. US 2010/0280495.

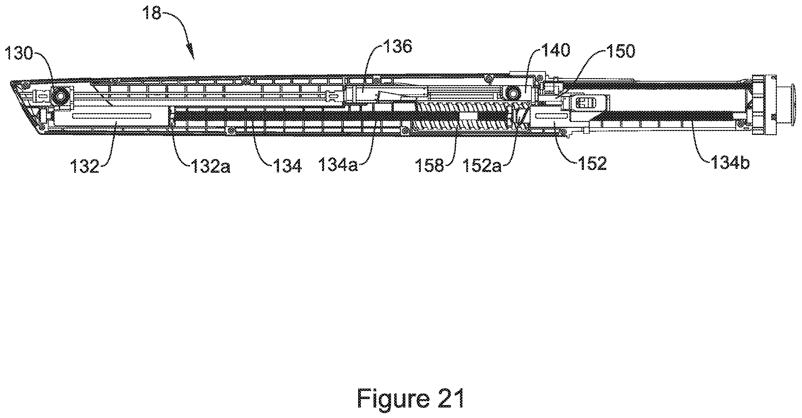

To help facilitate the coordinated movement, handle 18 may include a lost motion barrel 158. Lost motion barrel 158 is configured to engage carriages 132/152 and/or screws associated with carriages 132/152 at different times during the intervention to stop motion (e.g., create "lost motion" of the appropriate carriage). Figures 21-24 illustrate some of the coordinated motion achieved by handle 18. It should be noted that some elements of system 10 arc not shown in Figures 21-25 for clarity. For example, Figure 21 illustrates a first position or state for handle 18 where outer sheath 12 is extended distally relative to inner catheter 14 (and handle 18) so as to fully sheath (e.g., contain) implant 16. While in this position, sheath carriage 132 is positioned adjacent to the distal end of handle 18. In addition, a rod screw 152a associated with push-pull rod carriage 152 is extended distally from push-pull rod carriage 152 and positioned within lost motion barrel 158. Upon rotation of control knob 122 (e.g., in the clockwise direction), lead screw 134 begins to rotate. Rotation of lead screw 134 causes sheath carriage 132 to move along lead screw 134 in the proximal direction, resulting in proximal movement of outer sheath 12 (e.g., "unsheathing" implant 16). This initial rotation of lead screw 134 also causes rod screw 152a to rotate. This may be because, for example, a knob or projection (not shown) on rod screw 152a may be engaged with a helical thread disposed along the interior of lost motion barrel 158. However, because rod screw 152a is spaced from push-pull rod carriage 152, it does not exert a force onto push-pull rod carriage 152. Thus, initial motion of control knob 122 does not result in movement of push-pull rod carriage 152 and, instead, only results in translation of sheath carriage 132 and rotation (and translation) of rod screw 152a.

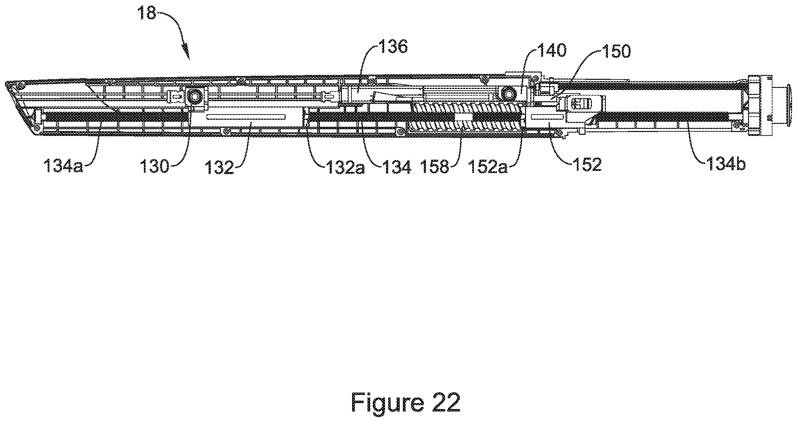

Eventually, rod screw 152a (e.g., the knob formed therein) reaches an essentially linear thread or pathway formed at the end of lost motion barrel 158. The linear thread allows rod screw 152a to translate along lead screw 134 to a position where rod screw 152a contacts (e.g., is threaded within and abuts) push-pull rod carriage 152. In doing so, rod screw 152a can contact and move proximally push-pull carriage 152. Accordingly, further rotation of lead screw 134 not only causes sheath carriage 132 to move proximally but also causes push-pull rod carriage 152 to move proximally as shown in Figure 22.

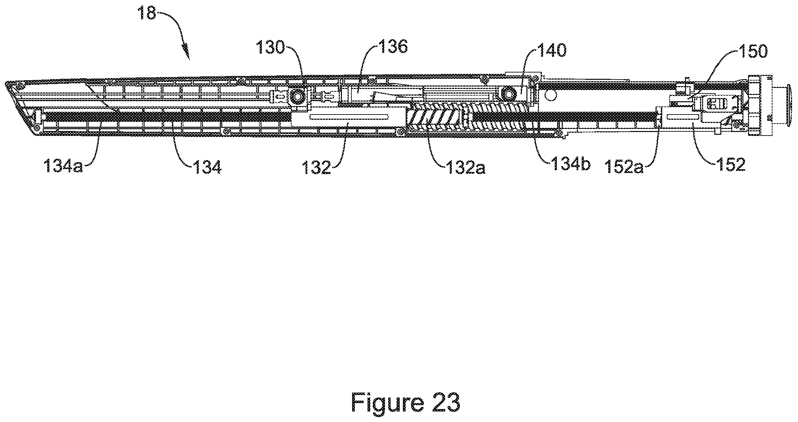

When sheath carriage 132 reaches lost motion barrel 158, a sheath carriage screw 132a of sheath carriage 132 enters lost motion barrel 158 as shown in Figure 23. This may occur in a manner similar to how rod screw 152a threads and unthreads with the helical thread formed along lost motion barrel 158. For example, while sheath carriage 132 is translating, sheath carriage screw 132a may follow an essentially linear thread or pathway formed along or adjacent to lost motion barrel 158. Upon reaching lost motion barrel 158, sheath carriage screw 132a (e.g., a knob or projection formed thereon) may shift into engagement with the helical thread within lost motion barrel 158 and rotate. This rotation "unthreads" sheath carriage screw 132a from sheath carriage 132. Accordingly, additional rotation of lead screw 134 results in continued proximal movement of push-pull rod carriage 152 while motion of sheath carriage 132 ceases.

In at least some embodiments, lead screw 134 has a plurality of portions, for example a first portion 134a and a second portion 134b, with a differing pitch to its thread. This may allow carriages 132/152 to travel at different rates along lead screw 134. For example, the pitch of lead screw 134 along which sheath carriage 132 translates may be generally more spaced or slanted than at positions adjacent to push-pull rod carriage 152. Accordingly, the coordinated movement of carriages 132/152 also may be configured so that sheath carriage 132 translates along lead screw 134 at a greater rate than push-pull rod carriage 152. Other configurations are contemplated where the above-mentioned configuration is reversed as well as further configurations where the pitch of lead screw 134 is essentially constant or includes a number of different pitch regions.

Sufficient proximal retraction of push-pull rod carriage 152, for example as shown in Figure 23, may result in push-pull rods 84 being sufficiently retracted so that posts 72 can engage and lock with buckles 76. When the clinician is satisfied that locking is complete (e.g., after verification via an appropriate visualization technique), the clinician may proximally retract pin release mandrel 92 in order to pull pins 88 from openings 98 and openings in push-pull rods 84 to release implant 16.

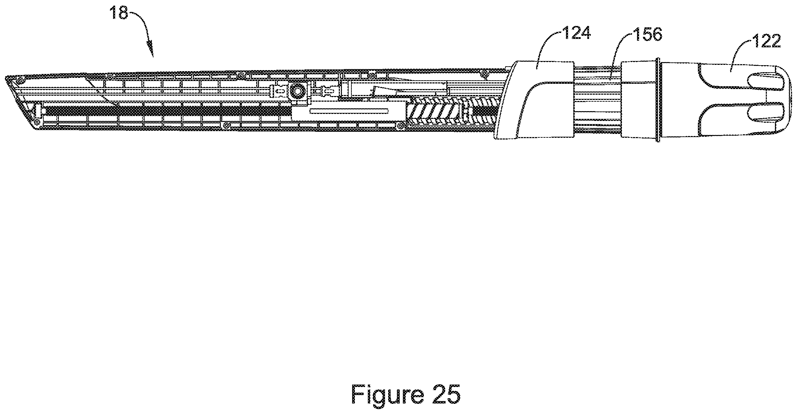

To initiate release of pins 88, door 124 may be slid distally along a collar 156 (which is positioned on handle 18) as shown in Figure 24. When door 124 is sufficiently advanced, door 124 and collar 156, together, can be rotated as shown in Figure 25. Push-pull rod carriage 152 may also include a radially-extending proximal flag member 164. In general, flag member 164 may be designed as a feature that can prevent collar 156 from being rotated earlier than desired (and, thus, prevent pins 88 from being pulled earlier than desired). For example, flag member 164 may be positioned within and follow a groove (not shown) along the interior of collar 156. While positioned within the groove, flag member 164 essentially forms a physical barrier that prevents collar 156 from rotating relative to handle housing 120. When push-pull rod carriage 152 is translated proximally to the back of handle housing 120 (e.g., when push-pull rods 84 are proximally retracted so as to lock posts 72 with buckles 76), flag member 164 exits the groove in collar 156. Accordingly, flag member 164 no longer impedes rotation of collar 156 and, as such, collar 156 can now be rotated to pull pins 88.

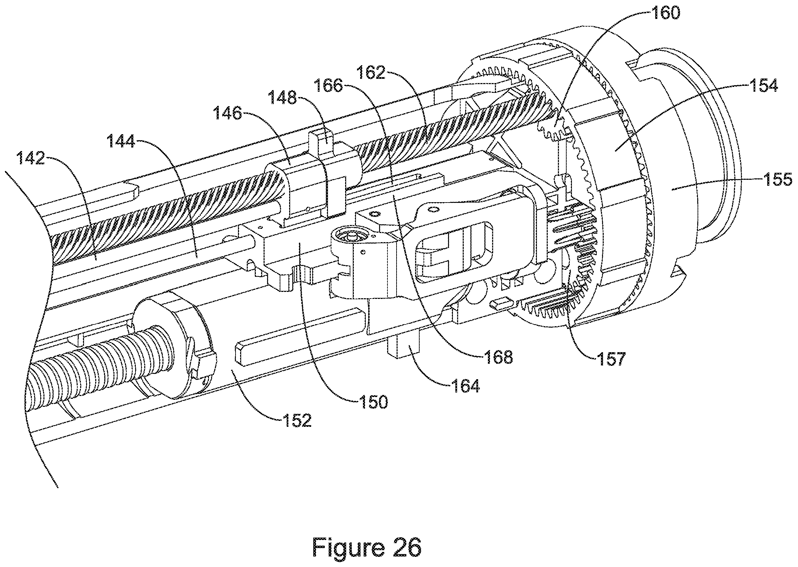

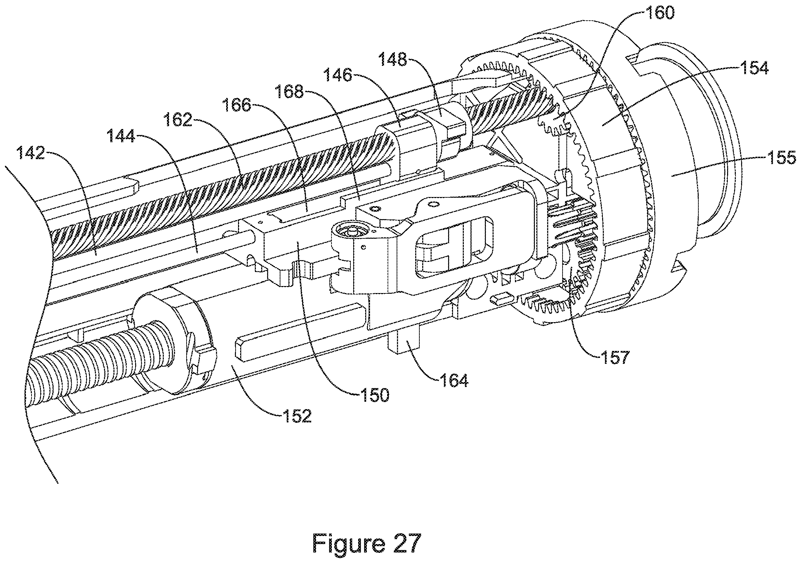

Collar 156, via ring 154, is associated with a gear 160 engaged with a secondary screw 162. Notches at a proximal end of collar 156 engage protrusions on ring 154 such that rotation of collar 156 causes corresponding rotation of ring 154 and thus secondary screw 162. The initial rotation of collar 156 is sufficient to rotate chock 148 (e.g., via a mechanical interaction between collar 156 and chock 148 that causes chock 148 to shift) from a first configuration where slider 146 (and, thus, pin release mandrel 92) is selectively locked to force limiter body 150, to a secondary configuration, which permits slider 146 to translate along secondary screw 162 as secondary screw 162 rotates, to proximally retract and pull pins 88 (e.g., via pin release mandrel 92). As seen in Figure 26, chock 148 in the first configuration engages a ridge 168 along a top portion of force limiter body 150 which forms a physical barrier that prevents proximal translation of slider 146 relative to force limiter body 150. When collar 156 is rotated to shift chock 148 into the secondary configuration, slider 146 can translate proximally within a groove 166 disposed in the top portion of force limiter body 150 (e.g., as seen in Figure 27), as collar 156 is rotated about the handle housing 120 to pull the pins 88 from the openings 98 and the openings in the distal ends of the push-pull rods 84. Once pins 88 have been removed, push-pull rods 84 may be withdrawn from implant 16, thereby deploying the implant at the target site (area of interest).

Following deployment of the implant 16, the control knob 122 may be rotated to move the sheath carriage 132 distally within the handle housing 120, thereby moving outer sheath 12 distally relative to inner catheter 14 and three-finger coupler 78 so as to cover or re-sheath the elements of the medical device system 10 disposed at the distal end. Medical device system 10 may then be removed from the patient's anatomy.

The materials that can be used for the various components of system 10 (and/or other systems disclosed herein) and the various tubular members disclosed herein may include those commonly associated with medical devices. For simplicity purposes, the following discussion makes reference to outer sheath 12 and/or inner catheter 14. However, this is not intended to limit the devices and methods described herein, as the discussion may be applied to other similar tubular members and/or components of tubular members or devices disclosed herein.

Outer sheath 12 and/or inner catheter 14 may be made from a metal, metal alloy, polymer (some examples of which arc disclosed below), a metal-polymer composite, ceramics, combinations thereof, and the like, or other suitable material. Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL® 625, UNS: N06022 such as HASTELLOY® C-22®, UNS: N10276 such as HASTELLOY® C276®, other HASTELLOY® alloys, and the like), nickel-copper alloys (e.g., UNS: N04400 such as MONEL® 400, NICKELVAC® 400, NICORROS® 400, and the like), nickel-cobalt-chromium-molybdenum alloys (e.g., UNS: R30035 such as MP35-N® and the like), nickel-molybdenum alloys (e.g., UNS: N10665 such as HASTELLOY® ALLOY B2®), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY®, PHYNOX®, and the like); platinum enriched stainless steel; titanium; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear that the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also can be distinguished based on its composition), which may accept only about 0.2 to 0.44 percent strain before plastically deforming.

In some embodiments, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Some examples of nickel titanium alloys are disclosed in U.S. Patent Nos. 5,238,004 and 6,508,803. Other suitable materials may include ULTANIUM™ (available from Neo-Metrics) and GUM METAL™ (available from Toyota). In some other embodiments, a superelastic alloy, for example a superelastic nitinol can be used to achieve desired properties.

In at least some embodiments, portions or all of outer sheath 12 and inner catheter 14 may also be doped with, made of, or otherwise include a radiopaque material. Radiopaque materials are understood to be materials capable of producing a relatively bright image on a fluoroscopy screen or another imaging technique during a medical procedure. This relatively bright image aids the user of system 10 in determining its location. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of system 10 to achieve the same result.

In some embodiments, a degree of Magnetic Resonance Imaging (MRI) compatibility is imparted into system 10. For example, outer sheath 12 and inner catheter 14, or portions thereof, may be made of a material that does not substantially distort the image and create substantial artifacts (e.g., gaps in the image). Certain ferromagnetic materials, for example, may not be suitable because they may create artifacts in an MRI image. Outer sheath 12 and inner catheter 14, or portions thereof, may also be made from a material that the MRI machine can image. Some materials that exhibit these characteristics include, for example, tungsten, cobalt-chromium-molybdenum alloys (e.g., UNS: R30003 such as ELGILOY®, PHYNOX®, and the like), nickel-cobalt-chromium-molybdenum alloys (e.g., UNS: R30035 such as MP35-N® and the like), nitinol, and the like, and others.

A sheath or covering (not shown) may be disposed over portions or all of outer sheath 12 and inner catheter 14 that may define a generally smooth outer surface for system 10. In other embodiments, however, such a sheath or covering may be absent from a portion of all of system 10, such that outer sheath 12 and inner catheter 14 may form an outer surface. The sheath may be made from a polymer or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN® available from DuPont), polyether block ester, polyurethane (for example, Polyurethane 85A), polypropylene (PP), polyvinylchloride (PVC), polyether-ester (for example, ARNITEL® available from DSM Engineering Plastics), ether or ester based copolymers (for example, butylene/poly(alkylene ether) phthalate and/or other polyester elastomers such as HYTREL® available from DuPont), polyamide (for example, DURETHAN® available from Bayer or CRISTAMID® available from Elf Atochem), elastomeric polyamides, block polyamide/ethers, polyether block amide (PEBA, for example available under the trade name PEBAX®), ethylene vinyl acetate copolymers (EVA), silicones, polyethylene (PE), Marlex high-density polyethylene, Marlex low-density polyethylene, linear low density polyethylene (for example REXELL®), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR®), polysulfone, nylon, nylon-12 (such as GRILAMID® available from EMS American Grilon), perfluoro(propyl vinyl ether) (PFA), ethylene vinyl alcohol, polyolefin, polystyrene, epoxy, polyvinylidene chloride (PVdC), poly(styrene-*b*-isobutylene-*b-*styrene) (for example, SIBS and/or SIBS 50A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

In some embodiments, the exterior surface of the system 10 (including, for example, the exterior surface of outer sheath 12 and inner catheter 14) may be sandblasted, beadblasted, sodium bicarbonate-blasted, electropolished, etc. In these as well as in some other embodiments, a coating, for example a lubricious, a hydrophilic, a protective, or other type of coating may be applied over portions or all of the sheath, or in embodiments without a sheath over portion of outer sheath 12 and inner catheter 14, or other portions of system 10. Alternatively, the sheath may comprise a lubricious, hydrophilic, protective, or other type of coating. Hydrophobic coatings such as fluoropolymers provide a dry lubricity which improves device handling and device exchanges. Lubricious coatings improve steerability and improve lesion crossing capability. Suitable lubricious polymers are well known in the art and may include silicone and the like, hydrophilic polymers such as high-density polyethylene (HDPE), polytetrafluoroethylene (PTFE), polyarylene oxides, polyvinylpyrolidones, polyvinylalcohols, hydroxy alkyl cellulosics, algins, saccharides, caprolactones, and the like, and mixtures and combinations thereof. Hydrophilic polymers may be blended among themselves or with formulated amounts of water insoluble compounds (including some polymers) to yield coatings with suitable lubricity, bonding, and solubility. Some other examples of such coatings and materials and methods used to create such coatings can be found in U.S. Patent Nos. 6,139,510 and 5,772,609.

The coating and/or sheath may be formed, for example, by coating, extrusion, co-extrusion, interrupted layer co-extrusion (ILC), or fusing several segments end-to-end. The layer may have a uniform stiffness or a gradual reduction in stiffness from the proximal end to the distal end thereof. The gradual reduction in stiffness may be continuous as by ILC or may be stepped as by fusing together separate extruded tubular segments. The outer layer may be impregnated with a radiopaque filler material to facilitate radiographic visualization. Those skilled in the art will recognize that these materials can vary widely.

Reference is made to the following documents: U.S. Patent Application Pub No. 2013/0123795A1, U.S. Patent Application Pub No. US 2013/0123898A1, U.S. Patent Application Pub No. 2013/0123912A1, U.S. Patent No. 9,131,926, U.S. Patent Application No. 2013/0123796A1, U.S. Patent No. 8,951,243, U.S. Patent Application Pub No. 2013/0158655A1, and U.S. Patent Application Pub No. 20130158653A1.

It should be understood that this disclosure is, in many respects, only illustrative. Changes may be made in details, particularly in matters of shape, size, and arrangement of steps. This may include, to the extent that it is appropriate, the use of any of the features of one example embodiment being used in other embodiments. The invention is, of course, defined in the language in which the appended claims are expressed.

## Claims

1. A medical device release system, comprising:
an elongated rod (92) having an enlarged distal end (94);
a ring element (90) slidingly disposed over the elongated rod (92), the ring element having a center channel (93) sized and shaped to receive the elongated rod, wherein the enlarged distal end (94) of the elongated rod is larger than an inner diameter of the center channel (93) preventing the ring element from disengaging from the enlarged distal end of the elongated rod; **characterized by**
two or more pins (88) each having a proximal end (89) attached to the ring element (90) and a distal end extending longitudinally and radially away from the ring element (90), the distal end of each of the two or more pins (88) configured for releasable connection to a medical device (16), wherein the proximal end (89) of each of the two or more pins (88) is enlarged, wherein the ring element (90) has two or more side channels (95), wherein each side channel (95) is sized to receive one of the two or more pins (88), wherein the enlarged proximal end (89) of each of the two or more pins (88) is larger than an inner diameter of the two or more side channels (95) of the ring element (90).

2. The medical device release system of claim 1, wherein the two or more pins (88) are welded to the ring element.

3. The medical device release system of claim 1 or 2, wherein each pin (88) is positioned within a respective side channel (95) in the ring element (90) with the enlarged proximal end (89) of each pin disposed adjacent a proximal end of the ring element, wherein each pin has a first bend (87) as it emerges from a distal end of the ring element, wherein a combination of the enlarged proximal end (89) of the pin and the first bend (87) holds the pin in a fixed axial position relative to the ring element.

4. The medical device release system of claim 3, wherein each pin (88) has a first section (91) disposed between the enlarged proximal end (89) and the first bend (87), a second section (97) extending from the first bend (87) to a second bend (85), and a third section (99) extending from the second bend (85) to the distal end of the pin.

5. The medical device release system of claim 4, wherein the second bend (85) is greater than 360 degrees.

6. The medical device release system of claim 4, wherein the second bend includes a spring.

7. The medical device release system of any of the preceding claims, wherein the two or more pins include three pins attached to the ring element.

8. The medical device release system of claim 7, wherein the three pins are equally spaced around the ring element.

9. A medical device delivery system, comprising:
an outer sheath;
an inner catheter disposed within the outer sheath, the inner catheter having a distal end;
an implant releasably coupled to the inner catheter; and
the implant release mechanism according to any of the preceding claims, wherein the elongated rod is slidingly disposed within the inner catheter.

10. A medical device delivery system, comprising:
a sheath having a distal end;
a coupler attached to the distal end of the sheath, the coupler including a plurality of coupling fingers;
an implant releasably coupled to the coupling fingers; and
the implant release mechanism according to any of claims 1 to 8, wherein the elongated rod is slidingly disposed within the sheath.

## Patentansprüche

1. Freigabesystem für eine medizinische Vorrichtung, umfassend:
eine längliche Stange (92) mit einem vergrößerten distalen Ende (94),
ein Ringelement (90), das verschiebbar über der länglichen Stange (92) angeordnet ist,
wobei das Ringelement einen mittleren Kanal (93) hat, der zur Aufnahme der länglichen Stange bemessen und geformt ist, wobei das vergrößerte distale Ende (94) der länglichen Stange größer als ein Innendurchmesser des mittleren Kanals (93) ist, wodurch verhindert wird, dass das Ringelement aus dem vergrößerten distalen Ende der länglichen Stange ausrückt, **gekennzeichnet durch**
zwei oder mehr Stifte (88), die jeweils ein proximales Ende (89), das an dem Ringelement (90) angebracht ist, und ein distales Ende haben, das sich in Längsrichtung und radial von dem Ringelement (90) weg erstreckt, wobei das distale Ende jedes der zwei oder mehr Stifte (88) zur freigebbaren Verbindung mit einer medizinischen Vorrichtung (16) ausgestaltet ist, wobei das proximale Ende (89) jedes der zwei oder mehr Stifte (88) vergrößert ist, wobei das Ringelement (90) zwei oder mehr Seitenkanäle (95) hat, wobei jeder Seitenkanal (95) zur Aufnahme eines der zwei oder mehr Stifte (88) bemessen ist, wobei das vergrößerte proximale Ende (89) jedes der zwei oder mehr Stifte (88) größer als ein Innendurchmesser der zwei oder mehr Seitenkanäle (95) des Ringelements (90) ist.

2. Freigabesystem für eine medizinische Vorrichtung nach Anspruch 1, wobei die zwei oder mehr Stifte (88) an das Ringelement geschweißt sind.

3. Freigabesystem für eine medizinische Vorrichtung nach Anspruch 1 oder 2, wobei jeder Stift (88) in einem jeweiligen Seitenkanal (95) in dem Ringelement (90) positioniert ist, wobei das vergrößerte proximale Ende (89) jedes Stifts einem proximalen Ende des Ringelements benachbart angeordnet ist, wobei jeder Stift eine erste Biegung (87) hat, wenn er aus einem distalen Ende des Ringelements austritt, wobei eine Kombination des vergrößerten proximalen Endes (89) des Stifts und der ersten Biegung (87) den Stift in einer festgelegten axialen Position bezüglich des Ringelements hält.

4. Freigabesystem für eine medizinische Vorrichtung nach Anspruch 3, wobei jeder Stift (88) einen ersten Abschnitt (91), der zwischen dem vergrößerten proximalen Ende (89) und der ersten Biegung (87) angeordnet ist, einen zweiten Abschnitt (97), der sich von der ersten Biegung (87) zu einer zweiten Biegung (85) erstreckt, und einen dritten Abschnitt (99), der sich von der zweiten Biegung (85) zu dem distalen Ende des Stifts erstreckt, aufweist.

5. Freigabesystem für eine medizinische Vorrichtung nach Anspruch 4, wobei die zweite Biegung (85) größer als 360 Grad ist.

6. Freigabesystem für eine medizinische Vorrichtung nach Anspruch 4, wobei die zweite Biegung eine Feder aufweist.

7. Freigabesystem für eine medizinische Vorrichtung nach einem der vorhergehenden Ansprüche, wobei die zwei oder mehr Stifte drei an dem Ringelement angebrachte Stifte aufweisen.

8. Freigabesystem für eine medizinische Vorrichtung nach Anspruch 7, wobei die drei Stifte gleichmäßig um das Ringelement herum beabstandet sind.

9. Zuführsystem für eine medizinische Vorrichtung, umfassend:
eine äußere Hülse,
einen in der äußeren Hülse angeordneten inneren Katheter, wobei der innere Katheter ein distales Ende hat,
ein freigebbar an den inneren Katheter gekoppeltes Implantat und
den Implantatfreigabemechanismus nach einem der vorhergehenden Ansprüche,
wobei die längliche Stange verschiebbar in dem inneren Katheter angeordnet ist.

10. Zuführsystem für eine medizinische Vorrichtung, umfassend:
eine Hülse mit einem distalen Ende,
einen Koppler, der an dem distalen Ende der Hülse angebracht ist, wobei der Koppler eine Vielzahl von Koppelfingern aufweist,
ein freigebbar an die Koppelfinger gekoppeltes Implantat und
den Implantatfreigabemechanismus nach einem der Ansprüche 1 bis 8, wobei die längliche Stange verschiebbar in der Hülse angeordnet ist.

## Revendications

1. Système de libération de dispositif médical, comprenant :
une tige allongée (92) comportant une extrémité distale élargie (94) ;
un élément annulaire (90) disposé de manière coulissante sur la tige allongée (92), l'élément annulaire comportant un canal central (93) présentant des dimensions et une forme adaptées à la réception de la tige allongée, l'extrémité distale élargie (94) de la tige allongée étant plus large qu'un diamètre intérieur du canal central (93), ceci empêchant l'élément annulaire de se désaccoupler de l'extrémité distale élargie de la tige allongée ; **caractérisé par**
au moins deux broches (88) ayant chacune une extrémité proximale (89) attachée à l'élément annulaire (90) et une extrémité distale s'étendant longitudinalement et radialement dans une direction s'éloignant de l'élément annulaire (90), l'extrémité distale de chacune des au moins deux broches (88) étant conçue à des fins de raccordement libérable avec un dispositif médical (16), l'extrémité proximale (89) de chacune des au moins deux broches (88) étant élargie, l'élément annulaire (90) comportant au moins deux canaux latéraux (95), chaque canal latéral (95) présentant des dimensions adaptées à la réception de l'une des au moins deux broches (88), l'extrémité proximale élargie (89) de chacune des au moins deux broches (88) étant plus large qu'un diamètre intérieur des au moins deux canaux latéraux (95) de l'élément annulaire (90).

2. Système de libération de dispositif médical selon la revendication 1, dans lequel les au moins deux broches (88) sont soudées à l'élément annulaire.

3. Système de libération de dispositif médical selon la revendication 1 ou 2, dans lequel chaque broche (88) est positionnée à l'intérieur d'un canal latéral respectif (95) dans l'élément annulaire (90), l'extrémité proximale élargie (89) de chaque broche étant disposée de manière adjacente à une extrémité proximale de l'élément annulaire, dans lequel chaque broche comporte une première courbure (87) à sa sortie d'une extrémité distale de l'élément annulaire, dans lequel une combinaison de l'extrémité proximale élargie (89) de la broche et de la première courbure (87) maintient la broche dans une position axiale fixe par rapport à l'élément annulaire.

4. Système de libération de dispositif médical selon la revendication 3, dans lequel chaque broche (88) comporte une première section (91) disposée entre l'extrémité proximale élargie (89) et la première courbure (87), une deuxième section (97) s'étendant de la première courbure (87) à une seconde courbure (85), et une troisième section (99) s'étendant de la seconde courbure (85) à l'extrémité distale de la broche.

5. Système de libération de dispositif médical selon la revendication 4, dans lequel la seconde courbure (85) fait plus de 360 degrés.

6. Système de libération de dispositif médical selon la revendication 4, dans lequel la seconde courbure comprend un ressort.

7. Système de libération de dispositif médical selon l'une quelconque des revendications précédentes, dans lequel les au moins deux broches comprennent trois broches attachées à l'élément annulaire.

8. Système de libération de dispositif médical selon la revendication 7, dans lequel les trois broches sont espacées de manière équidistante autour de l'élément annulaire.

9. Système de pose de dispositif médical comprenant :
une gaine extérieure ;
un cathéter intérieur disposé à l'intérieur de la gaine extérieure, le cathéter intérieur comportant une extrémité distale ;
un implant accouplé de manière libérable avec le cathéter intérieur ; et
le mécanisme de libération d'implant selon l'une quelconque des revendications précédentes, la tige allongée étant disposée de manière coulissante à l'intérieur du cathéter intérieur.

10. Système de pose de dispositif médical, comprenant :
une gaine comportant une extrémité distale ;
un dispositif d'accouplement attaché à l'extrémité distale de la gaine, le dispositif d'accouplement comprenant une pluralité de doigts d'accouplement ;
un implant accouplé de manière libérable avec les doigts d'accouplement ; et
le mécanisme de libération d'implant selon l'une quelconque des revendications 1 à 8, la tige allongée étant disposée de manière coulissante à l'intérieur de la gaine.
